(19) Europäisches Patentamt
European Patent Office
Office européen des brevets

(11) Publication number: **0 082 789**
**B1**

(12) **EUROPEAN PATENT SPECIFICATION**

(45) Date of publication of patent specification: **08.04.87**

(51) Int. Cl.[4]: **C 07 K 7/64,** A 61 K 37/02, A 61 K 39/00, G 01 N 33/53

(21) Application number: **82402351.9**

(22) Date of filing: **21.12.82**

# (54) Immunological composition and method for hepatitis B virus.

(30) Priority: **22.12.81 GB 8138629**

(43) Date of publication of application:
**29.06.83 Bulletin 83/26**

(45) Publication of the grant of the patent:
**08.04.87 Bulletin 87/15**

(84) Designated Contracting States:
**CH DE FR GB IT LI NL**

(56) References cited:
**EP-A-0 044 710**
**WO-A-81/00577**
**US-A-4 287 185**

**CHEMICAL ABSTRACTS, vol. 97, no. 17, 1982, page 505, no. 142821v, Columbus, Ohio, USA P.K. BHATNAGAR et al.: "Immune response to synthetic peptide analogs of hepatitis B surface antigen specific for the a determinant"**

**ANGEWANDTE CHEMIE, vol. 13, no. 1, 1974, pages 10-19, Verlag Chemie GmbH., DE. H. TSCHESCHE: "Biochemistry of natural proteinase inhibitors"**

(73) Proprietor: **Baylor College of Medicine**
**Texas Medical Center 1200 Moursund Avenue**
**Houston Texas 77030 (US)**

(72) Inventor: **Dreesman, Gordon Ronald**
**6704 Community Drive**
**Houston Texas 77005 (US)**
Inventor: **Sparrow, James Thomas**
**12119 Atwell**
**Houston Texas 77035 (US)**
Inventor: **Peterson, Darrell Lynn**
**4345 Roundhill Drive**
**Chesterfield Virginia 23832 (US)**
Inventor: **Hollinger, Frederick Blaine**
**2124 Watts**
**Houston Texas 77030 (US)**
Inventor: **Melnick, Joseph Louis**
**8838 Chatsworth**
**Houston Texas 77024 (US)**

(74) Representative: **Martin, Jean-Jacques et al**
**Cabinet REGIMBEAU 26, Avenue Kléber**
**F-75116 Paris (FR)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

(56) References cited:

**NATURE, vol. 295, no. 5845, 1982, pages 158-160, Macmillan Journals Ltd., Chesham, Bucks, GB. ks, GB. G.R. DREESMAN et al.: "Antibody to hepatitis B surface antigen after a single inoculation of uncoupled synthetic HBsAg peptides"**

**Description**

The invention described herein was made during the course of work under grants from the National Heart, Lung and Blood Institute, National Institutes of Health, and the United States Army Medical Research and Development Command

Prior art statement and background of the invention

The following is an explanation of the background of the present invention together with a listing which, in the opinion of the Applicants, sets forth the closest prior art of which the Applicants are aware. A concise explanation of the relevance of the more important items is included.

Viral hepatitis has assumed significant world-wide epidemic proportions. It is estimated that there are two hundred million carriers of hepatitis B virus ("HBV") world-wide. The development of a conventional vaccine has been hampered by the inability to grow hepatitis B virus in tissue culture. As a result, it has been necessary to produce hepatitis B subunit particle vaccines by isolating and purifying the 22 nm lipoprotein particles composed of hepatitis B surface antigen ("HBsAg") from plasmas of asymptomatic human carriers. However, such formalin- or heat-inactivated vaccines have the disadvantage of substantial expense and limited supply. In addition, such a source presents potential hazards in view of unknown factors that may be present in the plasma. Also, as high-risk populations are immunized, sources of plasma containing large quantities of HBsAg will become scarce.

Chemically synthesized vaccines to replace viral vaccines offer the advantage of precise biochemical characterization, exclusion of genetic material of viral origin, exclusion of host- or donor-derived substances, consistent potency, and the like. Ideally, such synthetic vaccines do not have irrelevant microbial antigenic determinants, proteins or other materials that might otherwise contaminate the essential immunogen and cause unwanted side effects.

The possibility of a synthetic peptide vaccine for HBV has been suggested in the past. The following is a list of references relating to such vaccines or processes with respect thereto and which will be referred to herein by the respective reference numbers:

1. McAuliffe, V. J., Purcell, R. H. & Gerin, J. L. Rev. Infect. Dis. 2, 470—492 (1980).
2. Rao, K. R. & Vyas, G. N. Nature New Biol. 241, 240—241 (1973).
3. Melnick, J. L., Dreesman, G. R. & Hollinger, F. B. J. Infect. Dis. 133, 210—229 (1976).
4. Anderer, F. A. Biochim. Biophys. Acta 71, 246—248 (1963).
5. Fearney, F. J., Leung, C. Y., Young, J. D. & Bejamini, E. Biochim. biophys. Acta 243, 509—514 (1971).
6. Langbeheim, H., Arnon, R. & Sela, M. Proc. Natn. Acad. Sci. U.S.A. 73, 4636—4640 (1976).
7. Tiollais, P., Charnay, P. & Vyas, G. N. Science 213, 406—411 (1981).
8a. Peterson, D. L. J. Biol. Chem. 256, 6975—6983 (1981).
8b. Gavilaives, F., Gonzalez-Ros, F. M. & Peterson, D. L. J. Biol. Chem. 257, 7770 (1982).
8c. Peterson, D. L. *In* Viral Hepatitis (Eds. W. Szmuness, H. J. Alter and J. E. Maynard). Franklin Institute Press, Philadelphia, p. 707 (1982).
9a. Lerner, R. A. et al Proc. Natn. Acad. Sci. U.S.A. 78, 3403—3407 (1981).
9b. Prince, A. M., Ikram, H. & Hopp, T. P. Proc. Natl. Acad. Sci. U.S.A. 79, 579 (1982).
9c. Bhatnagar, P. R., Papas, E., Blum, H. E. Milich, D. R., Nitecki, D., Karels, M. J. & Vyas, G. N. Proc. Natl. Acad. Sci. U.S.A. 79, 4400 (1982).
10. Chou, P. Y. & Fasman, G. D. Adv. Enzym. 47, 45—148 (1978).
11. Bull, H. B. & Breese, K. Archs Biochem. Biophys. 161, 665—670 (1975).
12. Atassi, M. Z. Immunochemistry 12, 423—438 (1975).
13. Merrifield, R. B. Adv. Enzym. 32, 221—296 (1969).
14. Edelstein, M. S., McNair, D. S. & Sparrow, J. T. in Peptides: Synthesis, Structure, Function (eds. Rich, D. H & Gross, E.) (Pierce Chemical Co., Rockford, pp. 217—220), 1981.
15. Sparrow, J. T. J. Org. Chem. 41, 1350—1353 (1976).
16. Atherton, E., Woolley, V. & Sheppard, R. C. JCS Chem. Commun., 970 (1981).
17. Mao, S. J. T., Sparrow, J. T., Gilliam, E. B., Gotto, A. M. & Jackson, R. L. Biochemistry 16, 4150—4156 (1977).
18. Felix, A. M., Jiminez, M. H., Wang, C. T. & Meinhofer, J. Int. J. Peptide Protein Res. 15, 342—354 (1980).
19. Dreesman, G. R., Hollinger, F. B., Sanchez, Y., Oefinger, P. & Melnick, J. L. Infect. Immun. 32, 62—67 (1981).
20. Sanchez, Y. et al Infect. Immun. 30, 728—733 (1980).
21. Dixon, W. J. & Brown, M. B. BMDP-79 (University of California Press, Berkeley, 1979).
22. Hollinger, F. B., Adam, E., Heiberg, D. & Melnick, J. L. in Viral Hepatitis (eds. Szmuness, W., Alter, H., & Maynard, J.) (Franklin Institute Press, Philadelphia, pp. 451—466, 1982).
23. Fudenberg, H. H. & Kunkel, H. G. J. Exp. Med. 106, 689—702 (1957).
24. Vyas, G. N. in Hepatitis B Vaccine (eds. Maupas, P. & Guesry, P.) (Elsevier, Amsterdam, 1981).
25. Hopp, T. P. & Woods, K. R. Proc. Natn. Acad. Sci. U.S.A. 78, 3824—3828 (1981).
26. Zuckerman, A. J. New Scient. 88, 167 (1980).

27. Skelly et al Nature 290, 51 (1981).
28. Hopp, T. P. Molec, Immun. 18, 869 (1981).
29. Charnay, P., Pourcel, C., Louise, A., Fritsch, A. and Tiollais, P. Proc. Natl. Acad. Sci. U.S.A. 76, 2222 (1979).
30. Valenzuela, P., Gray, P., Quiroga, M., Zaldivar, J., Goodman, A. M. and Rutter, W. J. Nature 280, 815 (1979).
31. Pasek, M., Golo, T., Gilbert W., Zink, B., Schaller, H., McKay, P., Leadbetter, G. and Murray, K. Nature 282, 575 (1979).
32. Allison, A. C., Buckland, F. E. and Andrewes, C. H. Virology 17, 171 (1962).
33. Carver, D. H. and Seto, D. S. Y. J. Virol. 2, 1482 (1968).
34. Hare, J. D. and Chan, J. C. Virology 34, 481 (1968).
35. Sukeno, N., Shirachi, R., Yamaguchi, J. and Ishida, N. J. Virol. 9, 182 (1972).
36. Vyas, G. N. Rao, K. R. and Ibrahim, A. B. Science 178, 1300 (1972).
37. Dreesman, G. R., Hollinger, F. B., McCombs, R. M. and Melnick, J. L. J. Gen. Virol. 19, 129 (1973).
38. Kohler, G. and Milstein C.: Continuous culture of fused cells secreting antibody of predefined specificity. Nature (London) 256:495—497 (1975).
39. Oudin, J., and Michel, M. 1963. Une nouvelle formed' allotypie des globulines y du serum de lapin aparement lie'e a le jonction et a la specificite anticoyss. C. R. Seanc. Soc., Paris 257:805.
40. Hollinger, F. B., Dreesman, G. R., Sanchez, Y., Cabral, G. A. and Melnick, J. L. Experimental hepatitis B polypeptide vaccine in chimpanzees. *In* Viral Hepatitis (eds. G. N. Vyas, S. N. Cohen and R. Schmid). Franklin Institute Press, Philadelphia, pp. 557—567, 1978.

Reference 1 relates to formalin-inactivated hepatitis B virus vaccines that have been produced in several laboratories. The sole source of material for these vaccines has been 22-nm lipoprotein particles composed of HBsAg and derived from plasma of persons chronically infected with HBV.

The possibility of a synthetic peptide vaccine for HBV was suggested in References 2 and 3 following studies carried out with tobacco mosaic virus (References 4 and 5) and MS-2 coliphage (Reference 6). This possibility became a reality when the amino acid sequence for HBsAg was deduced from the nucleotide sequence of the cloned HBV genome as reviewed in Reference 7. Reference 8 discloses that a portion of the major polypeptide derived from HBsAg, with a calculated molecular weight of 25,000 (P25), has been sequenced.

Hepatitis B polypeptide vaccines containing hepatitis B-specific antigenic determinants associated with a non-glycosylated polypeptide with a molecular weight in the range 22,000—24,000 and a glycosylated polypeptide with a molecular weight in the range 26,000—29,000 have been prepared and tested for safety, immunogenicity and protective efficacy in susceptible chimpanzees. (References 19, 26, 27 and 40). The non-glycosylated polypeptide and the glycosylated polypeptide referred to herein have molecular weights of 25,000 (P25) and 30,000 (GP30), respectively, as determined by their amino acid sequence deduced from the sequence of the cloned hepatitis B virus DNA genome. (Reference 7).

Previous studies have demonstrated that sulfhydryl groups and/or disulfide bonds play an important role in the tertiary structure of a number of animal viruses since biological activities such as infectivity and hemagglutination are destroyed by treatment with either alkylating (sulfhydryl binding) or reducing reagents (References 32—34). The differential effect of these different reagents is illustrated by the fact that a reducing agent such as dithiothreitol destroys the infectivity of many enterovirus, but these same viruses are unaffected by treatment with sulfhydryl binding reagents (References 32, 33). More specifically, it has been shown that the antigenic determinants (epitopes) associated with HBsAg are conformation-dependent. This was demonstrated in that reduction of the dislufide bonds contained in HBsAg and subsequent alkylation of the free thiol groups destroyed both the antigenic and the immunogenic activities associated with HBsAg (References 35—37).

Reference 28 describes a computerized analysis of the amino acid sequences of the HBsAg protein to predict an antigenic site determinant. An amino acid sequence of residues 138—149 was synthesized and examined for its ability to bind antibodies to a mixture of the *ad* and *ay* subtypes of HBsAg. the peptide bound 9% of the antibodies.

As reported in Reference 9a, thirteen peptides were chemically synthesized corresponding to amino acid sequences predicted from the nucleotide sequence for HBsAg. Seven out of the thirteen synthetic peptides elicited an anti-peptide response in rabbits inoculated with three or four doses of a series of peptides, each containing 14—15 amino acid residues, but only after covalent linkage of the peptides to a carrier protein. Activity also was found after multiple injections of a peptide containing 34 amino acids. In Reference 9b a synthetic peptide containing amino acid residues 138—149 of P25 was prepared. This peptide was reported to contain the *a* group reactivity as well as *d* subgroup reactivity. When this peptide was conjugated to human erythrocytes and injected into mice, it induced the formation of anti-HBs with or without the use of Freund's adjuvant. The investigation in Reference 9c prepared seven linear peptide analogues of HBsAg: 122—137, 128—134, 139—147, 139—158, 140—158, 145—158, and 150—158. For experimental immunization of rabbits the synthetic peptides were coupled to keyhole limpet hemocyanin. The investigators studied the antigenicity of each peptide analogue by serologic neutralization of human antibodies specific for the *a* determinant of HBsAg. Analogues 139—147, 139—158, and 140—158 showed antigenicity as well as induction of anti-HBsAg. The rabbit antibodies were inhibited with each of the three

peptide analogues and all serotypes of natural HBsAg, having only the *a* determinant in common. They reported that a linear form of peptide 122—137 and of peptide 128—134 covalent linked to a protein carrier failed to elicit production of anti-HBs in rabbits. (Reference 9c).

Two patent applications pertaining to the production of synthetic peptides with application to viral vaccines (with specific reference to HBsAg) have been noted. The first, by R. A. Lerner et al. (European Patent Application 044,710 filed 16 July 1981) describes the production of multiple synthetic peptides containing different amino acid sequences associated with the native P25 polypeptide derived from HBsAg. The Lerner et al. application differs from the present invention in two distinct areas. First, the Lerner et al. peptides are uniformly linear, and Lerner et al. made no attempt to produce tertiary conformation as described herein. Secondly, there was no attempt to purify the synthetic peptides before coupling to the protein carrier. Thirdly, Lerner et al. produced antibody to a peptide only after conjugation to a carrier. The antibody produced reacted with native HBsAg but no attempt was made to determine whether the linear peptides reacted with antibodies produced in human beings following a natural hepatitis B viral infection. As explained herein, the reaction of our circular peptide with human hepatitis B antibodies demonstrates the importance of cyclization of synthetic HBsAg peptides.

The second patent application by T. P. Hopp (European Patent Application No. 056,249 filed 8 January 1982) describes the use of a computer program analysis modified to predict the most hydrophilic region of viral and bacterial polypeptides. He has applied this to the synthesis of a peptide containing 6 amino acid residues corresponding to amino acids 141—147 of the P25 protein of HBsAg. Similar to the teachings of Lerner et al. mentioned above, Hopp's peptides are uniformly linear and no attempt has been made to construct the tertiary structure associated with the conformation dependent antigenic determinants of HBsAg. Neither Lerner et al. nor Hopp mentions cyclization of their peptides by formation of intrachain or interchain disulfide bonds as in the present invention.

None of the above references teaches or suggests cyclic peptides containing disulfide bonds in a hydrophilic region of the major viral polypeptide which, as will be described hereafter, elicits an antibody response in mice after a single injection without linkage to a protein carrier. The composition according to the present invention contains a determinant that elicits the production of antibody of similar specificity to that produced by immunization with the SDS-denatured linear virus P25 polypeptide described in References 19 and 40. While the synthetic peptides disclosed in Reference 9a have several amino acids (138—146) in common with the compositions according to the present invention and there are four overlapping carboxyl amino acids of the peptides, there are no other substantial similarities between the teachings of References 9a, 9b and 9c and the present invention.

A. H. Whitte et al. article, Angew Chem. internat. Edit. *13* (1974) 10—19 deals with biochemistry of natural proteinase inhibitors in all of which the reactive site is situated in a loop closed by a disulfide bridge. It does not relate to hepatitis B surface antigen and does not suggest that the immunogenicity of a subunit of said antigen can be enhanced by the formation of disulfide bonds.

U.S. Patent 4,287,185 describes polypeptides which are derived and characterized from wheat grain and which act with respect to animal cells at the S-phase of cell growth. It describes the use of such peptides, which contains an intrachain disulfide bridge, for detection of virus-infected cells. The amino acid sequence of these polypeptide is unrelated to any virus-specified amino acid sequence. Thus the described polypeptide cannot be used as a vaccine to prevent infection by any virus for it does not possess the amino acid sequences which must be present in the virus itself.

Summary of the invention

The present invention is directed to a new composition of matter, a composition for use in an immunizing preparation, a composition for eliciting production of antibody to HBsAg, a composition for eliciting antibody response to HBsAg, a method for determining whether the immunizing preparation (synthetic peptide) reacts with antibody in the host to a natural infection of the host, a sensitive method to assess the reactivity of the synthetic peptide containing native conformation with natural antibody using anti-idiotype reagents, a method to assess the reactivity of the synthetic peptide with monoclonal antibody prepared to the infectious agent, and a new diagnostic method. The composition is a cyclic peptide containing a disulfide bond in a hydrophilic region of the major viral polypeptide P25. The composition results in immunogenicity shown by the detection of antibody activity in mice seven days after a single injection of the peptide, disulfide cyclization being important in locking the secondary structure of a potent immunogen.

It is, therefore, an object of the present invention to provide a composition comprising a cyclic synthetic polypeptide comprising at least part of an antigenic sequence of the hepatitis B surface antigen and having a disulfide bond in a hydrophilic region of the peptide.

It is an object of the present invention to provide a composition comprising a cyclic synthetic peptide selected from an amino acid sequence of hepatitis B surface antigen containing at least two cysteine residues and having a disulfide bond between two cysteine amino acids.

Another object is to provide such a composition for use in any immunizing preparation specific to hepatitis B virus and/or for diagnostic use in connection therewith.

Another object of the present invention is the provision of such a composition for eliciting production of antibodies to hepatitis B surface antigen, and/or for eliciting antibody response to HbsAg.

Another object of the invention is the provision of a method for detecting the presence of antibody to hepatitis B surface antigen in a clinical specimen.

Another object of the present invention is the provision of a method to identify the relatedness of the cyclized synthetic peptide to antigenic determinants recognized by the host following a natural infection with the corresponding HBV.

Another object of the present invention is the provision of a method to identify antigenic determinants recognized by the host with the use of idiotype-anti-idiotype analysis.

Another object of the present invention is the provision of a method to identify the antigenic determinants recognized by the host with the use of monoclonal antibodies prepared to the native antigens of the infectious agent.

Yet another object of the present invention is the provision of a method for eliciting production of antibodies to HBsAg by introducing such a peptide into a host containing the antigen.

A still further object of the present invention is to provide a composition that will elicit protective antibodies against hepatitis B virus following immunization of humans.

Still other objects, features and advantages of the present invention will be apparent from the following description of the preferred embodiments of the invention, given for the purpose of disclosure and taken in conjunction with the accompanying drawings.

Brief description of the drawings

Figure 1 is an illustration of the generally accepted amino acid sequences of the major polypeptide derived from HBsAg with a calculated molecular weight of 25,000 (P25) as taught in Reference 7. The most hydrophilic regions as predicted by computer analysis (described in Reference 11) are single under lined.

Figure 2 is an illustration of the amino acid sequences of a cyclic polypeptide according to the present invention wherein the disulfide bond occurs at residues 124—137 and is indicated by the symbol =. The peptide illustrated in Figure 2 and described herein will be referred to as "peptide 1".

Figure 3 is an illustration of the amino acid sequences of another cyclic peptide according to the present invention similar to that of Figure 2 but containing five additional amino acid residues. The peptide illustrated in Figure 3 and described herein will be referred to as "peptide 2".

Figure 4 is an illustration of the amino sequence of a hydrophilic region of P25 derived from the various subtypes of HBsAg. The sequence for *ayw* as taught in reference 29 was deduced from the sequence of the cloned HBV DNA genome. Amino substitutions were noted at several positions for *adw* and *ayw* subtype polypeptides (References 30 and 31, respectively). The amino acid residue substitutions noted in References 8b and 8c were deduced from amino acid sequence analyses of purified preparations of P25 polypeptides.

Figure 5 graphically plots per cent inhibition of antibody to the *a* determinant of HBsAg by various concentrations of native HBsAg and cyclic peptide 1 to illustrate reactivity of a peptide as disclosed herein.

Figure 6 illustrates what the applicants herein theorize is the basic mechanism of the idiotype system herein.

Figure 7 graphically depicts inhibition of a human anti-HBs idiotype-anti-idiotype reaction $\bar{c}$ HBsAg subtypes *adw*, *ayw* and *adr*.

Detailed description of the preferred embodiment

Using techniques disclosed in Reference 10, a computer analysis was made of the amino acid sequence of P25 to predict the secondary structure of the molecule to permit selection of the amino acid residues containing specific HBsAg determinants. A portion of the P25 molecule illustrated in Figure 1 was sequenced as described in Reference 8a. The protein hydrophobicity was estimated using the amino acid assignments according to Reference 11. HBsAg was found to contain predominantly beta structure interrupted with a series of beta turns. Three highly hydrophobic regions were located between residues 1 and 42, 66 and 108, and 146 and 226, and two hydrophilic regions between residues 43 and 65, and 109 and 145. The latter hydrophilic regions are probably exposed on the intact particle and, consequently beta turns in these regions represent potentially exposed antigenic determinants. The region between amino acids 117 and 137 was chosen for synthesis inasmuch as a single cyclic disulfide bond could be formed between Cys 124 and Cys 137 as illustrated in Figures 2 and 3 if a serine was substituted for the cysteine at position 121 in the *ayw* sequence as taught in reference 8a. Thus, by locking the conformation of the potential beta turns at residues 129—132 and 135—137, a polypeptide more native in structure could be obtained. Lysine was substituted for Arg 122 to provide a handle for cross-linking to other proteins, if needed, before injection into animals. The selected peptides were synthesized by solid-phase methodology according to References 13 and 15 on a Schwarz Bioresearch synthesizer modified for computer control according to Reference 14. Butyloxycarbonyl-S-p-methoxybenzyl-L-cysteine was coupled to p-hydroxymethylphenyl-acetamidomethyl polystyrene using dicyclohexylcarbodiimide with a catalytic amount of 4-N,N-dimethylaminopyridine; the loading was 0.09 mM per g of resin. For the synthesis, the α-amino groups were protected with t-butyloxycarbonyl (t-BOC) and the side chain-protecting groups were as follows: benzyl ethers for the hydroxyl of serine and threonine, dichlorobenzyl ether for the phenolic hydroxyl of tyrosine and carbobenzoxy for the ε-amino group of lysine. Trifluoroacetic acid (40% in $CH_2Cl_2$) was used to remove the t-BOC protecting group, and the resulting salt was neutralized with N,N-diisopropylethylamine (5% in

$CH_2Cl_2$). Dicyclohexylcarbodiimide was used to couple the t-BOC amino acids; hydroxybenzotriazole was added to the coupling reaction of glutamine to suppress side chain dehydration. The specific steps of the synthesizer program are described in References 15 and 17.

The protecting groups were removed and the peptide was cleaved from the resin at 0°C with anhydrous hydrogen fluoride (HF) containing 10% anisole and 1% ethanedithiol as scavengers. The HF reagent was removed under vacuum at 0°C, and then the peptide was precipitated and washed with anhydrous ether. After extraction of the peptide from the resin with trifluoroacetic acid, the solvent was evaporated at 15°C and the peptide again precipitated with ether. The ether was decanted after centrifugation and the pellet dissolved in 1 M Tris/6 M guanidine hydrochloride; the pH of this solution was immediately adjusted to 8. The solution was desalted on a BioGel® P-2 column equilibrated in 0.1 M ammonium bicarbonate, and the peptide-containing fractions were pooled and lyophilized.

The cyclic disulfide was formed by oxidation of a dilute solution of the peptide with potassium ferricyanide according to the procedure of Reference 18. The solution was lyophilized, resuspended in ammonium bicarbonate and desalted on a BioGel® P-2 column, and the peptide further purified by ion-exchange chromatography at 4°C on a column of SP-Sephadex® equilibrated in 0.02 M $NaH_2PO_4$/6 M urea, pH 3.74. The material eluted with a linear gradient of 800 ml of 0.02 M $NaH_2PO_4$/6 M urea and 800 ml of 0.15 M NaCl/0.02 M $NaH_2PO_4$/6 M urea, pH 3.74. The peptide was located by measuring the absorbance at 275 nm.

After desalting on Bio-Gel® P-2 in ammonium bicarbonate and lyophilization, each peptide had the expected amino acid composition by amino acid analysis (Table 1) and eluted as a major peak on $C_{18}$-reverse-phase HPLC in a linear gradient of 1% triethyl ammonium phosphate, pH 3.2, and 2-propanol. The peptide was chromatographed on a column of Biogel® P-10 precalibrated with a series of unrelated cyclic and linear peptides. Peptide 1 eluted at a volume expected for the cyclic form with a molecular weight of 1708. The linear form eluted at the smaller volume expected for a linear peptide of this size.

TABLE 1

Amino acid composition of synthetic peptides

| Amino acid | Peptide 1 122—137 | Peptide 2 117—137 Ser 121 |
|---|---|---|
| Threonine* | 3.75(4) | 4.67(5) |
| Serine* | 1.93(2) | 3.55(4) |
| Glutamic acid | 1.06(1) | 1.06(1) |
| Proline | 0.85(1) | 1.92(2) |
| Glycine | 1.00(1) | 2.00(2) |
| Alanine | 0.99(1) | 1.08(1) |
| Half cystine** | — (2) | — (2) |
| Methionine | 1.87(2) | 1.87(2) |
| Tyrosine | 0.99(1) | 0.93(1) |
| Lysine | 0.93(1) | 0.92(1) |

Analyses were performed on a Beckman model 121 amino acid analyser after hydrolysis *in vacuo* for 24 h. at 110°C in 6 M HCl. The theoretical values are given in parentheses.

* Values are not corrected for destruction during hydrolysis.

** Half cystine was present in the amino acid analysis but was not integrated.

P25 was derived from a purified preparation of HBsAg after solubilization by heating at 100°C for 2 minutes in 0.5 M urea/1% SDS/1% 2-mercaptoethanol (References 19 and 40). The soluble polypeptides were fractionated by cylindrical polyacrylamide gel electrophoresis ("PAGE") in 10% gels. The SDS-denatured linear P25 polypeptide, which is known to contain 226 amino acid residues, was used as a control for immunogenicity studies.

The two resulting synthetic peptides (117—137 of Figure 3 and 122—137 of Figure 2) were incorporated into a series of adjuvant vehicles for immunization of mice. Equal volumes of immunogen and Freund's complete adjuvant ("FCA") were emulsified to give a final peptide concentration of 50 μg per 0.4 ml. The

alum-adsorbed preparations were prepared as described previously (References 19 and 40) with hydrated aluminium potassium sulfate, to give a final suspension containing 50 μg peptide per 0.4 ml. Large multilamellar liposomes were prepared from a mixture of dipalmitoylphosphatidylcholine cholesterol and dipalmitoylphosphatidic acid in a molar ratio of 2.0:1.5:0.2 respectively (per Reference 20). Dried lipid films were dispersed by vortexing in an aqueous medium at a phospholipid concentration of 20 mM. Liposomes were swollen in solutions containing either 100 μg of peptide per ml or 100 μg of peptide plus 1 mg $ml^{-1}$ muramyl dipeptide ("MDP"; Sigma). To ensure that comparable doses were given in each case, untrapped antigen was not removed. Previous studies reported in Reference 20 have indicated that about 60% of HBsAg materials were associated with liposomes prepared in these conditions. Final liposome concentrations were adjusted such that each animal received 6 mmol phospholipid and 25 μg peptide with or without 250 μg MDP in a volume of 0.3 ml.

Groups of six female BALB/c mice were immunized intraperitoneally with each of the above preparations. The mice were bled from the tail vein on days 7, 14 and 21. Samples were assayed at a serum dilution of 1:4 using a commercial solid-phase radio-immunoassay (AUSAB, Abbott, North Chicago). Anti-HBs concentrations are expressed in Table 2 as milli-International Units per millilitre (MIU $ml^{-1}$) based on the international reference preparation of anti-HBs (lot 26.1.77) provided by the International Laboratory for Biological Standards, Central Laboratory of the Netherlands Red Cross Transfusion Service. A computer nonlinear regression program, BmDP3R (per Reference 21), was used to describe the standard dose-response curve for the World Health Organization ("WHO") international reference preparation from which the potency of each of the samples was obtained.

TABLE 2

Anti-HBs concentrations in mice immunized with 25 μg synthetic HBsAg peptides 117—137 (Figure 3) and 122—137 (Figure 2)

| Immunizing preparation | Range of antibody concentration postinjection (mIU $ml^{-1}$) Day 7 | Day 14 | Day 21 |
|---|---|---|---|
| 117—137-FCA | 3.5—8.6(4/6)[a] | 3.2—10.3(5/6) | 2.6—13.9(5/6) |
| 122—137-FCA | 3.6—5.8(3/6) | 5.9—11.9(3/6) | 17.3—25.9(3/6) |
| 117—137-alum | 3.0—7.3(2/6) | 2.8—12.6(3/6) | 3.4—4.5(2/6) |
| 122—137-alum | 2.1—14.5(4/6) | 2.5—10.0(3/6) | 5.0—6.4(2/6) |
| 117—137-liposomes | 2.1—15.4(3/6) | 2.0—12.8(4/6) | — |
| 122—137-liposomes | 3.6—8.8(3/6) | 5.2—12.0(3/6) | 5.6—11.8(3/6) |
| 122—137-liposomes+MDP | 2.1—23.7(4/6) | 2.9—21.0(3/6) | 6.8—40.0(2/6) |
| P25 polypeptide-alum | 2.1—17.2(6/6) | 2.5—10.5(5/6) | 7.1—12.5(2/6) |

[a] Only positive antibody values are listed. Numbers in parentheses represent number of mice with an anti-HBs response over total number of mice in each group.

The results given in Table 2 show that (1) approximately 50% of the mice in each group produced detectable levels of antibody 7 days after inoculation with either the synthetic peptides or the P25 polypeptide; (2) the adjuvant vehicle—FCA, alum, liposomes, or liposomes with incorporation of MDP—did not significantly affect the primary antibody response; (3) in several groups the antibody response was greater on day 14, as shown by either the increased anti-HBs concentrations or the number of mice in each group which responded; (4) on day 21, the peak levels of antibody response in most groups of mice decreased; and (5) the antibody response in mice inoculated with purified SDS-denatured P25 was similar to that observed with the synthetic peptides.

When sera from each group of positive mice on days 7, 14 and 21, respectively, were pooled and fractionated by ultracentrifugation in 10—40% (w/v) preformed sucrose gradients (per Reference 23), IgG antibody was localized in the intermediate fractions and IgM was detected in the rapidly sedimenting fractions. Typical IgM and IgG responses were seen in each group, with one exception: there was no IgG in the group of mice injected with peptide 122—137 (peptide 1) emulsified with FCA.

Two procedures were used to enhance the immunogenic activity of cyclic peptide 1 (amino acid

residues 122—137) by methods that would be acceptable for field trials in humans. The first involved preparation of peptide micelles similar to those described previously for virus proteins. The second procedure involved the covalent linking of tetanus toxoid to peptide 1.

The peptide micelles were prepared by incubating 500 µg of peptide 1 suspended in 0.01 M Tris-HCl, 0.5 M NaCl, pH 7.3, with a final concentration of 2% Triton® X-100 at 37°C for 18 hours. Approximately 200,000 counts per minute (cpm) of $^{125}$I-labelled peptide 1 was added as tracer before the detergent treatment. The mixture was then layered on a Triton X-100 free 10—50% weight per volume (w/v) preformed sucrose gradient and centrifuged at 36,000 rpm for 72 hours in a Beckman® SW41 rotor. Two peaks of radioactivity were detected: a minor peak located in the first fraction (top of gradient), and a major band (containing >80% of the total label material) at approximately one-third the distance from the top of the gradient. Examination of the latter peak by electron microscopy revealed the presence of pleomorphic aggregates with diameters ranging from 40 to 80 nm. These aggregates sedimented more slowly and had a lower density (1.10 g/cm$^3$ in sucrose) than that observed when micelles were prepared from solubilized 22-nm HBsAg particles (1.19 g/cm$^3$ in sucrose). The peptide micelle preparation was dialyzed against 0.01 M Tris-HCl buffer, pH 7.2, for 18 hours at 4°C.

Peptide 1 and purified tetanus toxoid were conjugated using a modified carbodiimide method. Briefly, 500 µg of peptide was incubated with 5 mg of 1-ethyl-3-(3'-dimethylaminopropyl)carbodiimide-HCl (Sigma Chemical Co., St. Louis, Mo.) in 0.0125 M Na-phosphate buffer, pH 5.0, for 2 minutes at 22°C. The pH of the mixture was adjusted to 8.0 with 0.1 M NaOH. 3 ml of tetanus toxoid (Connaught Laboratories, Swiftwater, Pa.) containing 8 Lf/500 µg protein/ml was subsequently added and allowed to interact for 18 hours at 22°C. The resulting conjugate was dialyzed for 24 hours at 4°C against several changes of 0.1 M sodium phosphate buffered saline (0.15 M NaCl), pH 8.0.

The two preparations, peptide micelles and peptide-tetanus toxoid conjugate, were tested for immunogenicity in mice either as an aqueous suspension or after adsorption to alum gel. Alum precipitation of both vaccines was carried out as previously described. The final preparations of aqueous material and of alum gels were diluted in 0.15 M NaCl containing 0.01 M phosphate, pH 7.2, to contain 10 µg peptide/200 µl. For each vaccine, a group of 6 BALB/c mice received an intraperitoneal primary inoculation of 10 µg peptide. Mice were bled from the tail vein prior to inoculation and 7, 14, 21 and 32 days postinoculation. All sera were diluted 1:6 in 0.01 M PBS, pH 7.2, and tested for anti-HBs activity by the AUSAB solid phase radioimmunoassay. The results were reported as the ratio of the cpm of each serum sample (S) divided by the cpm obtained with a pool of the normal sera (N) obtained from the same group of mice before the primary inoculation. A 1:6 diluted serum sample with an SN value ⩾2.1 was considered positive.

The relative immunogenicity of each preparation, illustrated by the number of mice developing an anti-HBs response, is summarized in Table 3. Similar to earlier observations, a proportion of mice in each group responded with low levels of specific anti-HBs activity (S/N values ranging from 2.7 to 9) as early as 14 days after the primary inoculation. It should be noted that these mice received only 10 µg peptide 1 (versus 25 µg injected previously) and the starting serum dilution was 1:6 rather than 1:4. Bearing this in mind, the response was not better than had been noted after injection of BALB/c mice with liposome entrapped uncoupled peptide 1. The weakest immune response was noted in the group of mice immunized with peptide micelles in saline; only 1 of 6 mice produced a detectable level of antibody.

TABLE 3

Immunogenicity of 10 μg peptide 1 (amino acid residues 122—137) in micelle form or with a tetanus toxoid carrier

| | Immunogen | | | |
|---|---|---|---|---|
| | peptide micelles (saline) | peptide micelles (alum) | peptide-tetanus toxoid (saline) | peptide-tetanus toxoid (alum) |
| Preinoculation | 0/6* | 0/6 | 0/6 | 0/6 |
| Days after primary inoculation | | | | |
| 7 | 0/6 | 0/6 | 0/6 | 0/6 |
| 14 | 1/6 | 1/6 | 2/6 | 1/6 |
| 21 | 1/6 | 1/6 | 4/6 | 2/6 |
| 32 | 0/6 | 2/6 | 3/6 | 1/6 |
| Days after booster | | | | |
| 13 | 3/6 | 4/5 | 3/6 | 5/6 |
| 32 | 1/6 | 4/5 | 4/5 | 6/6 |

* No. of mice responding/No. of mice inoculated. All sera were tested at a 1:6 dilution using an AUSAB kit. Sera with S/N values ⩾2.1 were considered positive.

Thirty three days after the primary immunization, the mice were boostered with 50 μg of peptide in the respective vaccine preparations. The animals were bled at 13 and 32 days after booster, and the sera were tested for anti-HBs as described above. An anamnestic response was noted in all four groups of animals, in terms of percentage of responding animals (Table 3) and levels of antibody titers. As saline preparations, peptide micelles were a poor immunogen but the tetanus toxoid conjugate was a strong immunogen. Adsorption on alum gel greatly increased the immunogenicity of the micelle preparation, while only slightly enhancing the immune response to the peptide-tetanus toxoid conjugate. It is noteworthy that 80—100% of the mice possessed detectable levels of anti-HBs 32 days after the booster inoculation of peptide micelles adsorbed on alum gel and of peptide-tetanus toxoid in either saline solution or alum gel form (Table 3).

Fifty percent of the animals injected with peptide micelles adsorbed on alum and peptide-tetanus toxoid in saline produced antibodies that yielded S/N ratios of 10 or greater (at a serum dilution of 1:6) and 5 of 6 animals injected with peptide-tetanus toxoid-alum were positive at these levels (Table 4).

TABLE 4

Anti-HBs activity 32 days after a booster inoculation of mice with peptide 1 in micelle form or with a tetanus toxoid carrier

| Respective mouse number in each group | Immunogen | | | |
|---|---|---|---|---|
| | peptide micelles (saline) | peptide micelles (alum) | peptide-tetanus toxoid (saline) | peptide-tetanus toxoid (alum) |
| 1 | <2.1* | 14 | 20 | 123 |
| 2 | 2.7 | 22 | <2.1 | 27 |
| 3 | <2.1 | 76 | 153 | 96 |
| 4 | <2.1 | ND** | 18 | 10 |
| 5 | <2.1 | 7.5 | 3.4 | 25 |
| 6 | <2.1 | <2.1 | ND | 2.1 |

* S/N ratio determined using an AUSAB kit, at a 1:6 serum dilution. Sera with S/N values ≥2.1 were considered positive.

** ND=not done due to death of mouse.

The above observations demonstrate that a cyclic synthetic peptide analogous to amino acid positions 122 through 137 of P25 induces high levels of specific anti-HBs in mice. Although a primary response was noted in animals immunized with peptide 1 entrapped in liposomes, the anamnestic response was poor (data not shown). It appears that a peptide with a molecular weight of approximately 2,000 must be in an aggregated form, such as micelles, or must be linked to a protein carrier to efficiently recruit memory B cells.

Two tools have been developed and utilized to obtain data on the particular epitopes associated with the cyclic peptides. The first tool employed a panel of monoclonal anti-HBs antibodies to analyze the epitopes associated with peptide 1. The monoclonal antibody technique, originally developed by Kohler and Milstein (Reference 38), involves the establishment of a clone of lymphocytes, termed hybridomas, that produces homogenous antibody to a single epitope.

The epitope(s) associated with the synthetic cyclic peptide 1 (SP1) was initially analyzed using a panel of 14 anti-*a* monoclonal antibodies. None of these monoclonal preparations displayed any detectable reaction with either human serum albumin or human IgG. All reacted with purified particles of three different HBsAg subtypes: *adw*, *ayw* and *adr* (Tables 5 and 6). The anti-*a* specificity was further substantiated in that preincubation of each monoclonal antibody with purified HBsAg, subtype *adw* or *ayw*, decreased antibody binding to any HBsAg subtype on the solid phase by greater than 70%.

TABLE 5

Reaction of cyclic peptide 1 with anti-a monoclonal antibodies

| Monoclonal antibody | Coating HBsAg subtype | Counts bound without inhibitor | % Inhibition of binding* after blocking with: 80 µg cyclic peptide 1 | 6 µg HBsAg/*adw* |
|---|---|---|---|---|
| A-1 | *adw* | 2074 | 9.7 | 100 |
| | *ayw* | 1531 | 4.7 | 96 |
| | *adr* | 1343 | 6.1 | 95 |
| A-2 | *adw* | 1993 | 35.7 | 100 |
| | *ayw* | 1709 | 42.4 | 100 |
| | *adr* | 1555 | 87.7 | 100 |
| A-4 | *adw* | 1368 | 34.0 | 91 |
| | *ayw* | 689 | 63.6 | 100 |
| | *adr* | 1341 | 31.2 | 91 |
| A-6 | *adw* | 2809 | 0 | 97 |
| | *ayw* | 1944 | 0 | 100 |
| | *adr* | 1674 | 6.2 | 100 |
| A-7 | *adw* | 2647 | 0 | 95 |
| | *ayw* | 1989 | 2 | 100 |
| | *adr* | 1782 | 0 | 99 |
| A-8 | *adw* | 1042 | 0 | 100 |
| | *ayw* | 1762 | 10.5 | 100 |
| | *adr* | 1429 | 0 | 100 |
| A-10 | *adw* | 2272 | 0 | 100 |
| | *ayw* | 1561 | 0 | 98 |
| | *adr* | 1988 | 0 | 94 |
| A-11 | *adw* | 1267 | 0 | 100 |
| | *ayw* | 1734 | 1.8 | 100 |
| | *adr* | 1995 | 1.9 | 100 |
| A-12 | *adw* | 1438 | 67.0 | 100 |
| | *ayw* | 1987 | 66.8 | 100 |
| | *adr* | 1573 | 54.7 | 100 |
| A-13 | *adw* | 1407 | 47.2 | 100 |
| | *ayw* | 1742 | 51.8 | 80 |
| | *adr* | 1770 | 42.7 | 96 |
| A-14 | *adw* | 1078 | 15.2 | 100 |
| | *ayw* | 986 | 2.6 | 100 |
| | *adr* | 1231 | 13.5 | 91 |
| A-15 | *adw* | 1774 | 21.8 | 100 |
| | *ayw* | 1464 | 23.2 | 100 |
| | *adr* | 1389 | 32.1 | 90 |
| A-16 | *adw* | 878 | 0 | 100 |
| | *ayw* | 1979 | 0.2 | 98.5 |
| | *adr* | 668 | 0 | 90 |
| Hyb-1 | *adw* | 3353 | 0 | 100 |
| | *ayw* | 3730 | 5.0 | 99 |

* Measured by the ability of cyclic peptide 1 or HBsAg to inhibit the binding of each monoclonal antibody to a solid phase coated with various HBsAg. Values of ⩾15.0% were considered as positive.

TABLE 6
Reaction of cyclic and linear peptide 1 with anti-a monoclonal antibodies.

| Monoclonal antibody | Coating HBsAg subtype | Counts bound without | % Inhibition of binding* after blocking with: 80 µg cyclic peptide 1 | 80 µg linear peptide 1 | 6 µg HBsAg/*adw* | 6 µg HBsAg/*ayw* |
|---|---|---|---|---|---|---|
| A-2 | *adw* | 901 | 43 | 6 | 97 | 92 |
| A-4 | *adw* | 2567 | 31.8 | 28.8 | 100 | ND |
| A-12 | *adw* | 5162 | 41 | 0 | 99 | 99 |
| | *ayw* | 5728 | 57 | 0 | 99 | 100 |
| A-13 | *adw* | 2321 | 47 | 17 | 96 | 96 |
| | *ayw* | 2439 | 28 | 0 | 70 | 98 |

* Measured by the ability of peptide 1 or HBsAg to inhibit the binding of each monoclonal antibody to a solid phase coated with HBsAg.

The reactivity of the cyclic peptide 1 was examined further by incubating increasing quantities of the peptide with A-12 monoclonal antibody and subsequently testing the residual anti-*a* activity against HBsAg/*adw* by a micro-SPRIA test. As a control for this assay, the monoclonal antibody was also incubated with various concentrations of HBsAg, subtype *adw*. As shown in Figure 5, 6 µg of intact HBsAg particles abolished all anti-*a* activity of monoclonal A-12. Inhibition values ranged from 8 to 96% with HBsAg concentrations of 0.01 to 1 µg, respectively. Cyclic peptide 1 also blocked anti-*a* activity of A-12; however, compared on a weight basis to HBsAg, a 3000-fold excess of cyclic peptide 1 was necessary to achieve an equivalent degree of inhibition (Figure 5).

Based on the above experiments, the anti-*a* monoclonal antibodies were similarly tested against constant amounts of cyclic peptide 1 (80 µg/test) and HBsAg/*adw* (6 µg/test). As shown in Table 5, 80 µg of cyclic peptide 1 inhibited the reactivity of 6 of the 13 monoclonal antibodies between 13.5 and 87.7%. Individual monoclonal antibodies displayed different levels of inhibition: A-14 and A-15 were inhibited from 13 to 32%, whereas A-2, A-4, A-12 and A-13 were inhibited from 31 to 87%. These levels of inhibition were reproducible in repeat experiments. The group *a* specificity of the cyclic peptide 1 was also demonstrated in that preincubation with the peptide decreased the reactivity of an antibody with each of the three HBsAg subtypes used as coating antigen (Table 5). 6 µg of HBsAg/*adw* inhibited the anti-*a* reactivity of all 13 monoclonal preparations by greater than 90%.

The role of conformation in regard to the *a* antigenic activity of peptide 1 was demonstrated by retesting selected anti-*a* monoclonal preparations for their ability to react with linear peptide 1. Linear peptide 1 was obtained by reduction of the intrachain disulfide bond of cyclic peptide 1 and subsequent alkylation of the free thiol groups. Five monoclonal antibodies that failed to react with cyclic peptide 1 also did not react with the linear form. Two monoclonal antibodies that reacted with cyclic peptide 1 (A-2 and A-12) failed to exhibit a significant level of reactivity with linear peptide 1. The level of reactivity of A-13 with linear peptide 1 was markedely reduced. One exception was noted; monoclonal antibody A-4 was inhibited to a significant degree with both the cyclic and linear forms of the peptide (Table 6).

Three monoclonal antibodies previously characterized as reacting preferentially with the *y* subtype epitope (Y-3, Y-2, Hyb-2) were incubated with cyclic and linear peptide 1 and then tested for residual anti-HBs activity, utilizing HBsAg subtypes *adw*, *ayw* and *adr* as coating antigens. All three antibody preparations reacted with both cyclic and linear peptide 1 (Table 7). At high concentrations of Y-2 and Hyb-2 (dilutions of $10^{-1}$ and $10^{-4}$, respectively), inhibition was noted only with linear peptide 1, whereas at a 10-fold lower concentration of antibody each form of the peptide inhibited at similar levels.

TABLE 7
Reaction of cyclic and linear peptide 1 with anti-y and anti-w monoclonal antibodies

| Monoclonal antibody (dilution) | | Coating HBsAg subtype | Counts bound without inhibitor | % Inhibition of binding* after blocking with: | | | |
|---|---|---|---|---|---|---|---|
| | | | | 80 µg cyclic peptide 1 | 80 µg linear peptide 1 | 6 µg HBsAg/ *adw* | 6 µg HBsAg/ *ayw* |
| Y-3 | ($10^{-1}$) | *adw* | 166 | —** | — | — | — |
| | | *ayw* | 2554 | 17.5 | 23.8 | 0 | 98 |
| | | *adr* | 1677 | 6 | 3 | 0 | 100 |
| | ($10^{-2}$) | *ayw* | 870 | 31.2 | 31.0 | 0 | 95.4 |
| | | *adr* | 472 | 0 | 0 | 0 | 100 |
| Y-2 | ($10^{-1}$) | *adw* | 234 | — | — | — | — |
| | | *ayw* | 2358 | 4 | 27 | 4.2 | 93 |
| | | *adr* | 161 | — | — | — | — |
| | ($10^{-2}$) | *ayw* | 1939 | 37.5 | 23.5 | 6.9 | 98.9 |
| | ($10^{-3}$) | *ayw* | 735 | 46.8 | 40.4 | 1.8 | 100 |
| Hyb-2 | ($10^{-4}$) | *adw* | 43 | — | — | — | — |
| | | *ayw* | 1576 | 1.3 | 20 | 0 | 100 |
| | | *adr* | 2203 | 1.2 | 3.5 | 0 | 100 |
| | ($10^{-5}$) | *ayw* | 730 | 24 | 20.4 | 7.2 | 97.9 |
| | | *adr* | 719 | 0 | 0 | 0 | 100 |
| W-1 | | *adw* | 1278 | 0.8 | ND*** | 92 | ND |
| | | *ayw* | 1879 | 0.3 | ND | 87 | ND |
| | | *adr* | 258 | — | — | — | — |

* Measured by the ability of peptide 1 or HBsAg to inhibit the binding of each monoclonal antibody to a solid phase coated with various HBsAg subtypes.
** (—) indicates that these monoclonal antibodies failed to react with the respective coating HBsAg subtype and consequently inhibition could not be tested.
*** ND=not done.

It was noteworthy that two of the three anti-*y* preparations (Y-3 and Hyb-2) reacted with both *ayw* and *adr* coating antigens in the absence of inhibitors and that intact *ayw* particles blocked completely their reactivity with these two coating HBsAg subtypes (Table 7). However, linear and cyclic SP1 blocked only the reactivity with the *ayw* coating antigen.

The final analysis was performed with a monoclonal antibody that recognized the *w* subtype epitope. No inhibition of reactivity was observed upon incubation of this antibody with cyclic peptide 1 (Table 7).

These studies demonstrate that the group *a* cross-reactivity associated with HBsAg contains 2 or more distinct antigenic determinants and that the peptide contains one of these epitopes. The demonstration that a conformation-dependent group *a* epitope is associated with peptide 1 is important since a series of observations, both in humans and in experimentally infected chimpanzees, has demonstrated that antibody to the group *a* antigenic determinant produces immunity to all HBV subtypes; *adw, adr, ayw, ayr.* In addition, peptide 1 also contains a sequential *y* epitope.

The second immunochemical tool used to determine the ability of peptide 1 to elicit protective antibody resided with the idiotypic specificity of the anti-HBs antibodies produced by HBV-infected humans. The basic concept of the idiotypic system is illustrated in Figure 6. The region associated with the variable region ($V_L$ and $V_H$) of the immunoglobulin (Ig) molecule and which contains the antigen-combining site has been defined by Oudin (Reference 39) as the idiotype of that Ig molecule. An anti-idiotype reagent is produced by injection of a second species, such as rabbits, with specifically purified antibody, e.g., human anti-HBs. The resulting rabbit antiserum is extensively adsorbed with normal human Ig negative for anti-HBs reactivity. The anti-idiotype antiserum (with _^_ regions) thus recognizes only that region of the Ig molecule (shown as --o--) which specifically reacts with HBsAg. It has been found that humans infected with HBV generate anti-HBs which contains a common idiotype (CId). Thus, it is possible that the CId associated with human anti-HBs antibodies may be blocked from reacting with a specific anti-Id reagent by prior incubation with either HBsAg or with peptide 1 if the peptide is antigenically related to HBsAg. (See

Figure 6B for experimental design). The CId was found to be associated with the *a* group specificity in that purified 22-nm particles of *adw, ayw,* and *adr* specificities inhibited the CId-anti-idiotype reaction to the same degree (Figure 7). Both peptide 1 and peptide 2 efficiently inhibited the reaction. Inhibition was also obtained with a nondenatured HBsAg polypeptide (P25-GP30) micelle preparation (Table 8). The major role of conformation was emphasized in that reduction of the disulfide bond and alkylation of the free thio groups not only produced a linear peptide but also destroyed its capacity to block the CId-anti-idiotypic reaction (Table 8). Similarly, SDS-denatured preparations of P25 and GP30 also only partially inhibited the reaction providing further evidence that secondary structure is important in producing a potent immunogen.

In another series of experiments designed to further support our claims that the cyclic peptide 1 is antigenically related to antigenic determinants associated with HBsAg which elicit production of anti-HBs in humans, anti-HBs monoclonal antibodies were examined for the ability to inhibit the binding of the common idiotype to its respective anti-idiotype antiserum. Monoclonal antibodies that reacted with cyclic peptide 1 also were found to partially inhibit the common anti-HBs idiotype-anti-idiotype reaction (Table 9). Conversely, no inhibition was noted using monoclonal antibodies that failed to react with cyclic peptide 1. Thus, antibody preparations A-2, A-4, A-12 and Y-2 inhibited the idiotype-anti-idiotype reaction by greater than 22%, while A-16 and W-2 failed to demonstrate a significant level of inhibition. Mouse ascites fluid containing monoclonal antibodies with specificity for herpes simplex virus also failed to inhibit the common anti-HBs idio-type-anti-idiotype reaction, indicating that a 20% level of inhibition was not due to nonspecific reactivity.

TABLE 8

Percent inhibition of the common anti-HBs idiotype binding its anti-idiotype antiserum by different concentrations of various inhibitors

| Inhibitor | Concentration (μg) | Inhibition |
|---|---|---|
| Native HBsAg-derived polypeptide | 7.5 | 63 |
| | 3.75 | 45 |
| | 0.75 | 21 |
| | 0.375 | 18 |
| Native HBsAg-derived polypeptide, reduced and alkylated | 7.5 | 2 |
| | 0.75 | 0 |
| | 0.075 | 0 |
| SDS (denatured P25 and GP30) | 25.0 | 9—13 |
| | 2.5 | 2—5 |
| Synthetic peptide 1 | 250 | 63 |
| | 25 | 33 |
| | 2.5 | 30 |
| Synthetic peptide 1, reduced and alkylated | 25 | 1 |
| | 2.5 | 0 |
| Human serum albumin | 20.0 | 0 |
| | 5.0 | 0 |

TABLE 9

Inhibition pattern of monoclonal antibodies in association with cyclic peptide 1 or with a CHBs idiotype

| Monoclonal antibody | Epitope specificity | % Inhibition | |
|---|---|---|---|
| | | Cyclic peptide 1 | Common anti-HBs idiotype** |
| A-2 | *a* | 36—88 | 24 |
| A-4 | *a* | 31—64 | 25 |
| A-12 | *a* | 55—67 | 26 |
| A-16 | *a* | 0 | 0 |
| Y-2 | *y* | 40—47 | 22 |
| W-1 | *w* | 0 | 0 |

* The cyclic peptide was utilized to inhibit the reaction between the monoclonal antibody and HBsAg-coated wells.

** The monoclonal antibody was utilized to inhibit the common anti-HBs idiotype-anti-idiotype reaction.

The premise that cyclic peptide 1 contains an epitope similar to that which is recognized by the host following a natural infection by HBV is supported by the following study. A chimpanzee which had developed anti-HBs in response to an HBV infection, was inoculated with a conjugate of peptide 1 covalently linked to tetanus toxoid. Four weeks post-inoculation, the anti-HBs titer rose to 1:6560 from a base line level of 1:1160 as determined by radio-immunoassay (AUSAB). Thus, a booster response was noted to an epitope for which the host had prior immunological memory.

Several methods are available to optimize the conjugation protocols for coupling a synthetic peptide to a carrier protein. For instance (1) peptide 1 may be conjugated in varying molar ratios to tetanus toxoid through the free carboxyl group of the cysteine residue at position 137; (2) peptide 1 also may be conjugated in varying molar ratios to tetanus toxoid through the ε-amino group of the lysine residue at position 122; and (3) polymers of cyclic peptide 1 may be prepared by linking the position 137 carboxyl group to the ε-amino group of lysine 122.

Peptide 1 has been used as a diagnostic model for the detection of anti-HBs antibodies by cross-linking the peptide to polylysine using the carbodiimide procedure described above. This conjugate was used to coat micro wells of a 96-well polystyrene plate. Each micro-titer well was incubated with a variety of serum dilutions containing anti-HBs. Specific reaction of these antibodies was detected by the addition of specific $^{125}$I-labelled antiglobulin reagents. The results indicate that this protocol provides a highly sensitive and specific method for the detection of anti-HBs antibody. It follows that cyclic synthetic HBsAg peptides attached to a detectable probe can be used in competitive binding assays to detect HBsAg in clinical specimens similar to methods commonly used to measure small peptide hormone reactants.

Early work with a plant virus model using tobacco mosaic virus (References 4 and 5) and the bacterial virus model using MS-2 coliphage (Reference 6) has clearly demonstrated the immense potential of preparing immunologically active synthetic peptide analogues to amino acid sequences found in an animal virus protein. The present results indicate that amino acid residues localized in the major HBsAg polypeptide, P25, between residues 117 and 137—or more specifically, 122 and 137—contain a determinant that elicits the production of antibody of similar specificity to that produced following immunization with the SDS-denatured HBsAg P25 polypeptide as reported in References 19 and 40. These results are similar to those reported recently in Reference 9 which reports preparation of a series of linear synthetic peptides containing segments of the P25 HBsAg polypeptide. Four of the peptides (2—16, 22—35, 48—81 and 95—109) reported in Reference 9 elicited the production of antibody in rabbits which reacted with HBsAg. Synthetic peptides of residues 134—146 and 138—149 (References 24 and 25 respectively) of P25 also have been prepared. These peptides, which have several amino acids (138—146) in common, were both antigenically active in that each reacted with preparations containing specific anti-HBs activity, and they blocked antibody reactivity of sera of animals immunized with intact HBsAg. Linear peptides containing various overlapping amino acid residues in positions 138—158, covalently linked to carriers, have induced production of low levels of anti-HBs antibody in experimental animals (References 9b and 9c). The amino acid sequences of the immunogenic synthetic peptides according to the present invention reported in Table 2 as compared with the above studies have nothing in common. In fact, Reference 9c teaches that linear peptides 122—137 and 128—134 failed to elicit production of anti-HBs activity.

Whereas, the prior art (specifically, Reference 9a) teaches eliciting an antibody response to smaller peptides (e.g., having 14—15 residues), such response occurred only after repeated inoculation of covalently attached material to a carrier protein. In the present invention, antibody response to each of peptides 1 and 2 cyclized by disulfide bonds illustrated in Figures 2 and 3, respectively, occurs without using a carrier protein. It will be understood, however, that the incorporation of various adjuvant vehicles and/or protein carriers may increase the immunogenicity of the synthetic peptides according to the present invention.

The present invention, therefore, is well adapted to carry out the objects and attain the ends and advantages mentioned as well as others inherent therein. While a presently preferred embodiment of the invention has been given for the purpose of disclosure, numerous changes in compositions and steps of the methods will be readily apparent to those skilled in the art and which are encompassed within the spirit of the invention and the scope of the appended claims.

**Claims**

1. A composition of matter comprising a cyclic synthetic peptide comprising at least part of an antigenic sequence of the hepatitis B surface antigen and having a disulfide bond in a hydrophilic region of said cyclic peptide.

2. A composition of matter comprising a cyclic synthetic peptide selected from an amino acid sequence of hepatitis B surface antigen containing at least two cysteine residues and having a disulfide bond between two cysteine amino acids.

3. The composition of matter of Claim 2 wherein the peptide is one selected from a group consisting of a molecule having 16 amino acids and a molecule having 21 amino acids.

4. The composition of matter of Claim 2 wherein the disulfide bond is in the region of residues 122—137.

5. The composition of matter of Claim 2 wherein the disulfide bond is in the region of residues 117—137.

6. The composition of matter of Claim 2 wherein the cyclic peptide includes the following amino acid sequence:

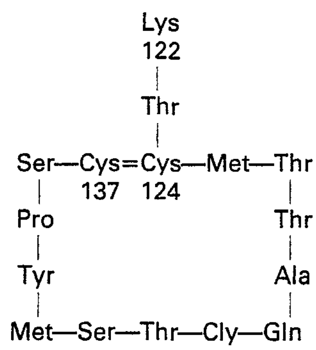

7. The composition of matter of Claim 2 wherein the cyclic peptide includes the following amino acid sequence:

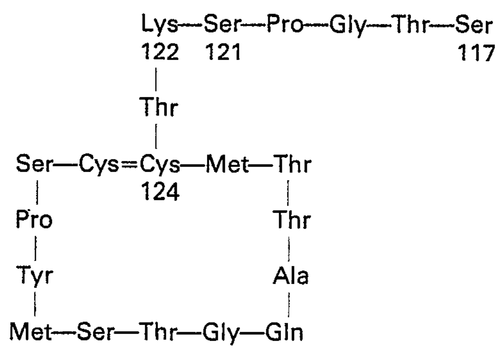

8. A composition for use as an immunizing preparation specific to hepatitis B virus comprising a cyclic synthetic peptide selected from an amino acid sequence of hepatitis B surface antigen containing at least two cysteine residues and having a disulfide bond between two cysteine amino acids.

9. A composition for eliciting production of antibody to hepatitis B surface antigen comprising a cyclic synthetic peptide selected from an amino acid sequence of hepatitis B surface antigen containing at least two cysteine residues and having a disulfide bond between two cysteine amino acids.

10. A composition for eliciting antibody response to hepatitis B surface antigen comprising a cyclic synthetic peptide selected from an amino acid sequence of hepatitis B surface antigen containing at least two cysteine residues and having a disulfide bond between two cysteine residues in the region of residues selected from the group consisting of 117—137 and 122—137.

11. The composition of Claim 10 wherein the cyclic peptide consists of 16 amino acid residues.

12. The composition of Claim 10 wherein the cyclic peptide consists of 21 amino acid residues.

13. The composition of Claim 11 wherein the cyclic peptide includes the following amino acid sequence:

```
              Lys
              122
               |
              Thr
               |
Ser—Cys=Cys—Met—Thr
 |   137  124          |
Pro                   Thr
 |                     |
Tyr                   Ala
 |                     |
Met—Ser—Thr—Gly—Gln
```

14. The composition of Claim 12 wherein the cyclic peptide includes the following amino acid sequence:

```
              Lys—Ser—Pro—Gly—Thr—Ser
              122  121                    117
               |
              Thr
               |
Ser—Cys=Cys—Met—Thr
 |        124          |
Pro                   Thr
 |                     |
Tyr                   Ala
 |                     |
Met—Ser—Thr—Gly—Gln
```

15. A composition for eliciting production of antibody to hepatitis B surface antigen comprising a carrier and a cyclic synthetic peptide having an amino acid sequence comprising at least part of an antigenic sequence of the hepatitis B surface antigen and having a disulfide bond in a hydrophilic region of said peptide.

16. A composition for eliciting antibody response to hepatitis B surface antigen, comprising a carrier and a cyclic synthetic peptide selected from an amino acid sequence of hepatitis B surface antigen containing at least two cysteine residues and having a disulfide bond between two cysteine amino acids.

17. The composition of Claim 16 wherein the cyclic polypeptide consists of 16 amino acid residues.

18. The composition of Claim 16 wherein the cyclic peptide consists of 21 amino acid residues.

19. The composition of Claim 17 wherein the cyclic peptide includes the following amino acid sequence:

```
              Lys
              122
               |
              Thr
               |
Ser—Cys=Cys—Met—Thr
 |   137  124          |
Pro                   Thr
 |                     |
Tyr                   Ala
 |                     |
Met—Ser—Thr—Gly—Gln
```

20. The composition of Claim 18 wherein the cyclic peptide includes the following amino acid sequence:

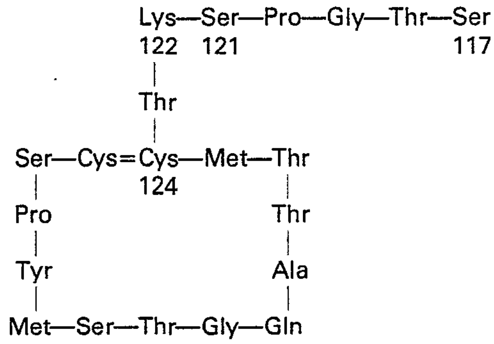

21. The composition of Claims 19 or 20 wherein the cyclic peptide is combined with an adjuvant vehicle.

22. The composition of Claims 19 or 20 wherein the adjuvant vehicle is selected from the group consisting of alum, liposomes or micelles.

23. A method for detecting the presence of antibody to hepatitis B surface antigen in a clinical specimen comprising adding said specimen to a cyclic synthetic peptide on a solid phase support, wherein the peptide is selected from an amino acid sequence of hepatitis B surface antigen containing at least two cysteine residues and having a disulfide bond between two cysteine amino acids.

24. The method of Claim 23 wherein the peptide is one selected from a group consisting of a molecule having 16 amino acids and a molecule having 21 amino acids.

25. The method of Claim 23 wherein the disulfide bond is in the region of residues 122—137.

26. The method of Claim 23 wherein the disulfide bond is in the region of residues 117—137.

27. The method of Claim 23 wherein the cyclic peptide includes the following amino acid sequence:

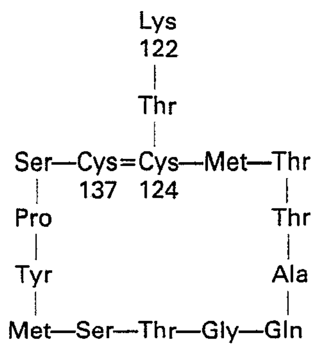

28. A method for the identification of the native antigenic determinants (epitopes) associated with an infectious agent through the competition of synthetic peptide of Claim 1 or 2 and anti-idiotype reagents produced to the common idiotype of naturally acquired antibody produced by the host to the infections agent.

29. The method of Claim 28 wherein the epitope associated with the synthetic peptide can be characterized with the use of monoclonal antibodies prepared to the antigen associated with the infectious agent.

**Patentansprüche**

1. Stoffzusammensetzung, welche ein cyclisches, synthetisches Peptid umfasst, das wenigstens Teil einer antigenen Sequenz des Hepatitis-B-Oberflächenantigens umfasst und eine Disulfidbindung in einer hydrophilen Region dieses cyclischen Peptids aufweist.

2. Stoffzusammensetzung, welche ein cyclisches, synthetisches Peptid, ausgewählt aus einer Aminosäurensequenz von Hepatitis-B-Oberflächenantigen, welche wenigstens zwei Cysteinreste enthält und eine Disulfidbindung zwischen zwei Cysteinaminosäuren aufweist, umfasst.

3. Stoffzusammensetzung nach Anspruch 2, worin das Peptid ein aus einer Gruppe, welche aus einem Molekül mit 16 Aminosäuren und einem Molekül mit 21 Aminosäuren besteht, ausgewähltes ist.

4. Stoffzusammensetzung nach Anspruch 2, worin die Disulfidbindung im Bereich der Reste 122—137 auftritt.

5. Stoffzusammensetzung nach Anspruch 2, worin die Disulfidbindung im Bereich der Reste 117—137 auftritt.

6. Stoffzusammensetzung nach Anspruch 2, worin das cyclische Peptid die nachstehende Aminosäurensequenz einschliesst:

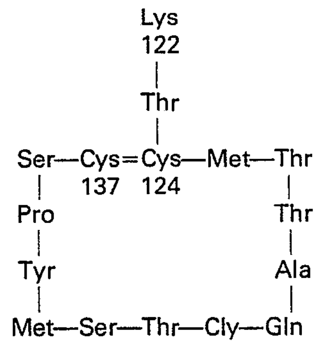

7. Stoffzusammensetzung nach Anspruch 2, worin das cyclische Peptid die nachstehende Aminosäurensequenz einschliesst:

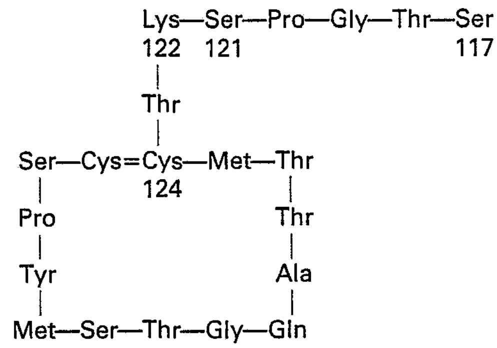

8. Zusammensetzung zur Verwendung als auf das Hepatits-B-Virus spezifische immunisierende Zubereitung, welche ein cyclisches, synthetisches Peptid, ausgewählt aus einer Aminosäurensequenz von Hepatitis-B-Oberflächenantigen, welche wenigstens zwei Cysteinreste enthält und eine Disulfidbindung zwischen Cysteinaminosäuren aufweist, umfasst.

9. Zusammensetzung zur Anregung der Produktion von Antikörpern auf Hepatitis-B-Oberflächenantigen, welche ein cyclisches, synthetisches Peptid, ausgewählt aus einer Aminosäurensequenz von Hepatitis-B-Oberflächenantigen, welche wenigstens zwei Cysteinreste enthält und eine Disulfidbindung zwischen zwei Cysteinaminosäuren aufweist, umfasst.

10. Zusammensetzung zur Anregung der Antikörper-Response auf Hepatitis-B-Oberflächenantigen, welche ein cyclisches, synthetisches Peptid, ausgewählt aus einer Aminosäurensequenz von Hepatitis-B-Oberflächenantigen, welche wenigstens zwei Cysteinreste enthält und eine Disulfidbindung zwischen zwei Cysteinresten in der Region der Reste, ausgewählt aus der Gruppe bestehend aus 117—137 und 122—137 aufweist, umfasst.

11. Zusammensetzung nach Anspruch 10, worin das cyclische Peptid aus 16 Aminosäureresten besteht.

12. Zusammensetzung nach Anspruch 11, worin das cyclische Peptid aus 21 Aminosäureresten besteht.

13. Zusammensetzung nach Anspruch 11, worin das cyclische Peptid die nachstehende Aminosäurensequenz einschliesst:

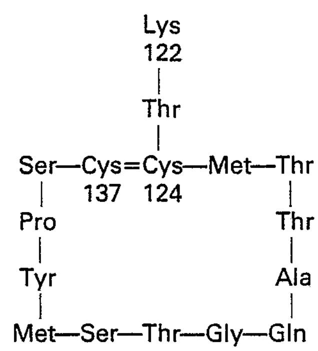

14. Zusammensetzung nach Anspruch 12, worin das cyclische Peptid die nachstehende Aminosäurensequenz einschliesst:

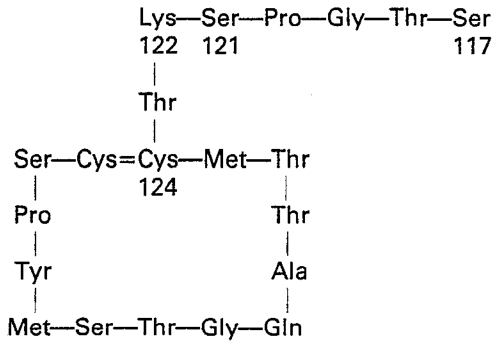

15. Zusammensetzung zur Anregung der Produktion von Antikörpern auf Hepatitis-B-Oberflächenantigen, welche einen Träger und ein cyclisches, synthetisches Peptid mit einer Aminosäurensequenz, welche wenigstens einen Teil einer antigenen Sequenz des Hepatitis-B-Oberflächenantigens umfasst, und mit einer Disulfidbindung in einer hydrophilen Region dieses Peptids umfasst.

16. Zusammensetzung zur Anregung der Antikörper-Response auf Hepatitis-B-Oberflächenantigen, welche einen Träger und ein cyclisches, synthetisches Peptid, ausgewählt aus einer Aminosäurensequenz von Hepatitis-B-Oberflächenantigen, welche wenigstens zwei Cysteinreste enthält und eine Disulfidbindung zwischen zwei Cysteinaminosäuren aufweist, umfaßt.

17. Zusammensetzung nach Anspruch 16, worin das cyclische Polypeptid aus 16 Aminosäureresten besteht.

18. Zusammensetzung nach Anspruch 16, worin das cyclische Peptid aus 21 Aminosäureresten besteht.

19. Zusammensetzung nach Anspruch 17, worin das cyclische Peptid die nachstehende Aminosäurensequenz einschliesst:

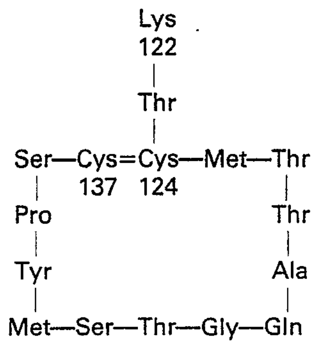

20. Zusammensetzung nach Anspruch 18, worin das cyclische Peptid die nachstehende Aminosäurensequenz einschliesst:

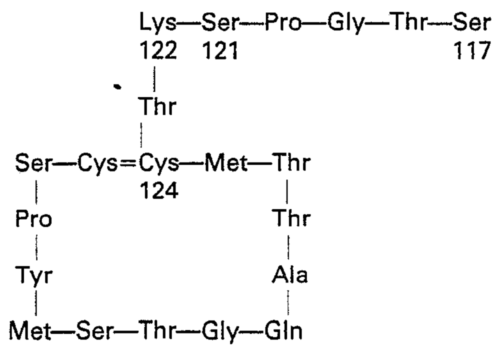

21. Zusammensetzung nach Ansprüchen 19 oder 20, worin das cyclische Peptid mit einem Adjuvansvehikel kombiniert wird.

22. Zusammensetzung nach Ansprüchen 19 oder 20, worin das Adjuvansvehikel aus der Gruppe, bestehend aus Alaun, Liposomen oder Micellen, ausgewählt wird.

23. Verfahren zum Nachweis der Gegenwart von Antikörpern auf Hepatitis-B-Oberflächenantigen in einer klinischen Probe durch Zugabe dieser Probe zu einem cyclischen, synthetischen Peptid auf einem Träger mit fester Phase, worin das Peptid ausgewählt wird aus einer Aminosäurensequenz von Hepatitis-B-Oberflächenantigen, welche wenigstens zwei Cysteinreste enthält und eine Disulfidbindung zwischen zwei Cysteinaminosäuren aufweist.

24. Verfahren nach Anspruch 23, worin das Peptid ein aus einer Gruppe, bestehend aus einem Molekül mit 16 Aminosäuren und einem Molekül mit 21 Aminosäuren, ausgewähltes ist.

25. Verfahren nach Anspruch 23, worin die Disulfidbindung in der Region der Reste 122—137 auftritt.

26. Verfahren nach Anspruch 23, worin die Disulfidbindung in der Region der Reste 117—137 auftritt.

27. Verfahren nach Anspruch 23, worin das cyclische Peptid die nachstehende Aminosäurensequenz einschliesst:

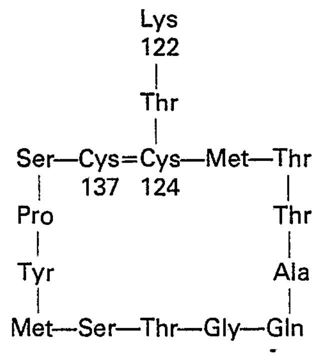

28. Verfahren zur Identifizierung der mit einem infektiösen Mittel assoziierten natürlichen antigenen Determinanten (Epitopen) durch Konkurrenz von synthetischem Peptid nach Anspruch 1 oder 2 und anti-Idiotypen Reagentien, welche auf den gewöhnlichen Idiotyp von natürlich erworbenem, vom Wirt des infektiösen Mittels produziertem Antikörper gebildet sind.

29. Verfahren nach Anspruch 28, worin der mit dem synthetischen Peptid assoziierte Epitop durch die Verwendung von zu dem mit dem infektiösen Mittel assoziierten Antigen hergestellten monoclonalen Antikörpern charakterisiert werden kann.

**Revendications**

1. Une composition comprenant un peptide synthétique cyclique comprenant au moins une partie d'une séquence antigénique de l'antigène de surface de l'hépatite B et ayant une liaison disulfure dans une région hydrophile dudit peptide cyclique.

2. Une composition comprenant un peptide synthétique choisi parmi une séquence d'amino-acides de l'antigène de surface de l'hépatite B contenant au moins deux restes de cystéine et ayant une liaison disulfure entre deux amino-acides cystéine.

3. La composition de la revendication 2 dans laquelle le peptide est choisi dans un groupe constitué d'une molécule ayant 16 amino-acides et d'une molécule ayant 21 amino-acides.

4. La composition de la revendication 2 dans laquelle la liaison disulfure est dans la région des restes 122—137.

5. La composition de la revendication 2 dans laquelle la liaison disulfure est dans la région des restes 117—137.

6. La composition de la revendication 2 dans laquelle le peptide cyclique comprend la séquence d'aminoacides suivante:

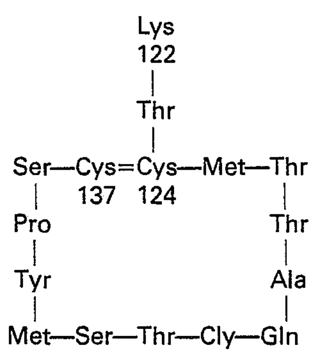

7. La composition de la revendication 2 dans laquelle le peptide cyclique comprend la séquence d'amino-acides suivante:

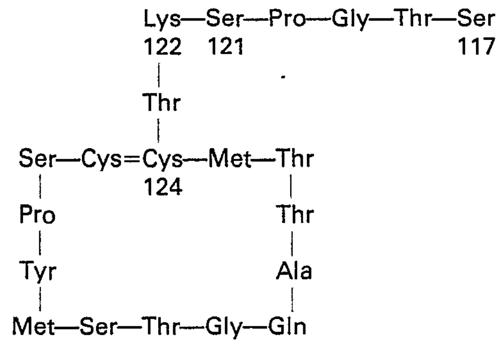

8. Une composition pour l'emploi comme préparation immunisante spécifique du virus de l'hépatite B comprenant un peptide synthétique cyclique choisi parmi une séquence d'amino-acides de l'antigène de surface de l'hépatite B contenant au moins deux restes de cystéine et ayant une liaison disulfure entre deux amino-acides cystéine.

9. Une composition pour provoquer la production d'un anticorps contre l'antigène de surface de l'hépatite B comprenant un peptide synthétique cyclique choisi parmi une séquence d'amino-acides de l'antigène de surface de l'hépatite B contenant au moins deux restes de cystéine et ayant une liaison disulfure entre deux aminoacides cystéine.

10. Une composition pour provoquer une réponse immunitaire contre l'antigène de surface de l'hépatite B comprenant un peptide synthétique cyclique choisi parmi une séquence d'amino-acides de l'antigène de surface de l'hépatite B contenant au moins deux restes de cystéine et ayant une liaison disulfure entre deux restes de cystéine dans la région des restes choisie dans le groupe constitué de 117—137 et 122—137.

11. La composition de la revendication 10 dans laquelle le peptide cyclique est constitué de 16 restes d'amino-acides.

12. La composition de la revendication 10 dans laquelle le peptide cyclique est constitué de 21 restes d'amino-acides.

13. La composition de la revendication 11 dans laquelle le peptide cyclique comprend la séquence d'amino-acides suivante:

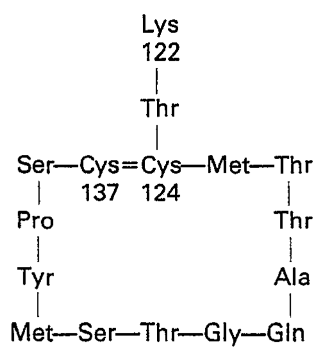

14. La composition de la revendication 12 dans laquelle le peptide cyclique comprend la séquence d'amino-acides suivante:

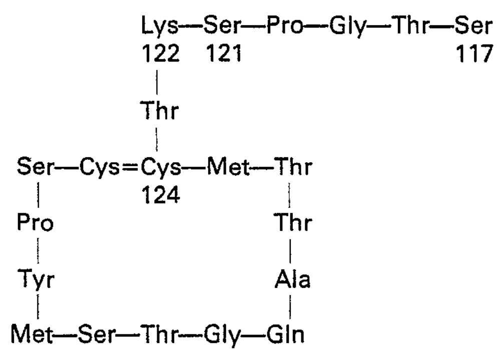

15. Une composition pour provoquer la production d'un anticorps contre l'antigène de surface de l'hépatite B comprenant un support et un peptide synthétique cyclique ayant une séquence d'amino-acides comprenant au moins une partie d'une séquence antigénique de l'antigène de surface de l'hépatite B et ayant une liaison disulfure dans une région hydrophile dudit peptide.

16. Une composition pour provoquer une réponse immunitaire contre l'antigène de surface de l'hépatite B comprenant un support et un peptide synthétique cyclique choisi parmi une séquence d'amino-acides de l'antigène de surface de l'hépatite B contenant au moins deux restes de cystéine et ayant une liaison disulfure entre deux amino-acides cystéine.

17. La composition de la revendication 16 dans laquelle le polypeptide cyclique est constitué de 16 restes d'amino-acides.

18. La composition de la revendication 16 dans laquelle le peptide cyclique est constitué de 21 restes d'amino-acides.

19. La composition de la revendication 17 dans laquelle le peptide cyclique comprend la séquence d'amino-acides suivante:

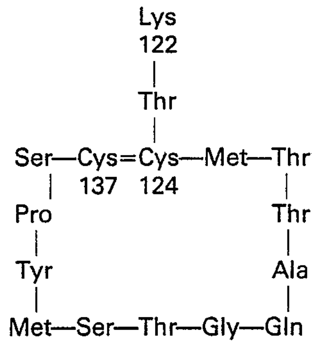

20. La composition de la revendication 18 dans laquelle le peptide cyclique comprend la séquence d'amino-acides suivante:

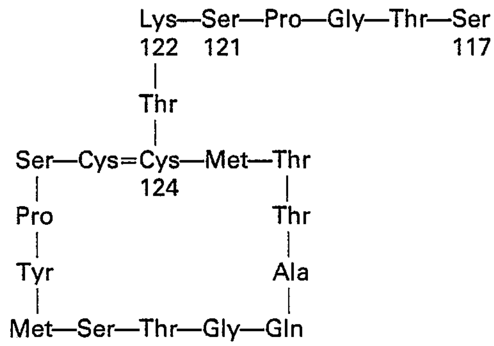

21. La composition de la revendication 19 ou 20 dans laquelle le peptide cyclique est combiné à un véhicule adjuvant.

22. La composition de la revendication 19 ou 20 dans laquelle le véhicule adjuvant est choisi dans le groupe constitué par l'alun, des liposomes ou des micelles.

23. Un procédé pour la détection de la présence d'un anticorps contre l'antigène de surface de l'hépatite B dans un échantillon clinique comprenant l'addition dudit échantillon à un peptide synthétique cyclique sur un support en phase solide, dans lequel le peptide est choisi parmi une séquence d'amino-acides de l'antigène de surface de l'hépatite B contenant au moins deux restes de cystéine et ayant une liaison disulfure entre deux amino-acides cystéine.

24. Le procédé de la revendication 23 dans lequel le peptide est choisi dans le groupe constitué par une molécule ayant 16 amino-acides et une molécule ayant 21 amino-acides.

25. Le procédé de la revendication 23 dans lequel la liaison disulfure est dans la région des restes 122—137.

26. Le procédé de la revendication 23 dans lequel la liaison disulfure est dans la région des restes 117—137.

27. Le procédé de la revendication 23 dans lequel le peptide cyclique comprend la séquence d'amino-acides suivante:

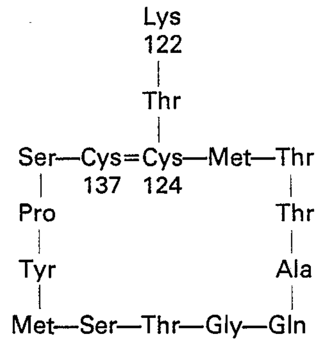

28. Un procédé pour l'identification des déterminants antigéniques natifs (épitopes) associés à un agent infectieux par la compétition d'un peptide synthétique des revendications 1 ou 2 et de réactifs antiidiotype produits contre l'idiotype commun de l'anticorps acquis naturellement produit par l'hôte contre l'agent infectieux.

29. Le procédé de la revendication 28 dans lequel l'épitope associé au peptide synthétique peut être caractérisé par l'emploi d'anticorps monoclonaux préparés contre l'antigène associé à l'agent infectieux.

FIGURE 1

Met - Glu - Asn - Ile - Thr - Ser - Gly - Phe - Leu - Gly (10) - Pro - Leu - Leu - Val - Leu -

-Gln - Ala - Gly - Phe - Phe (20) - Leu - Leu - Thr - Arg - Ile - Leu - Thr - Ile - Pro - Gln (30) -

-Ser - Leu - Asp - Ser - Trp - Trp - Thr - Ser - Leu - Asn (40) - Phe - Leu - Gly - Gly - Thr -

-Thr - Val - Cys - Leu - Gly (50) - Gln - Asn - Ser - Gln - Ser - Pro - Thr - Ser - Asn - His (60) -

-Ser - Pro - Thr - Ser - Cys - Pro - Pro - Thr - Cys - Pro (70) - Gly - Tyr - Arg - Trp - Met -

-Cys (80) - Leu - Arg - Arg - Phe - Ile - Ile - Phe - Leu - Phe - Ile - Leu - Leu - Leu - Cys (90) -

-Leu - Ile - Phe - Leu - Leu - Val - Leu - Leu - Asp - Tyr (100) - Glu - Gly - Met - Leu - Pro -

-Val - Cys - Pro - Leu - Ile (110) - Pro - Gly - Ser - Ser - Thr - Thr - Ser - Thr - Gly - Pro (120) -

-Cys - Arg - Thr - Cys - Met - Thr - Thr - Ala - Gln - Gly (130) - Thr - Ser - Met - Tyr - Pro -

-Ser - Cys - Cys - Cys - Thr (140) - Lys - Pro - Ser - Asp - Gly - Asn - Cys - Thr - Cys - Ile (150) -

-Pro - Ile - Pro - Ser - Ser - Trp - Ala - Phe - Gly - Lys (160) - Phe - Leu - Trp - Glu - Trp -

-Ala - Ser - Ala - Arg - Phe (170) - Ser - Trp - Leu - Ser - Leu - Leu - Val - Pro - Phe - Val (180) -

-Gln - Trp - Phe - Val - Gly - Leu - Ser - Pro - Thr - Val (190) - Trp - Leu - Ser - Val - Ile -

-Trp - Met - Met - Trp - Tyr (200) - Trp - Gly - Pro - Ser - Leu - Tyr - Ser - Ile - Leu - Ser (210) -

-Pro - Phe - Leu - Pro - Leu - Leu - Pro - Ile - Phe - Phe (220) - Cys - Leu - Trp - Val - Tyr - I

FIGURE 2

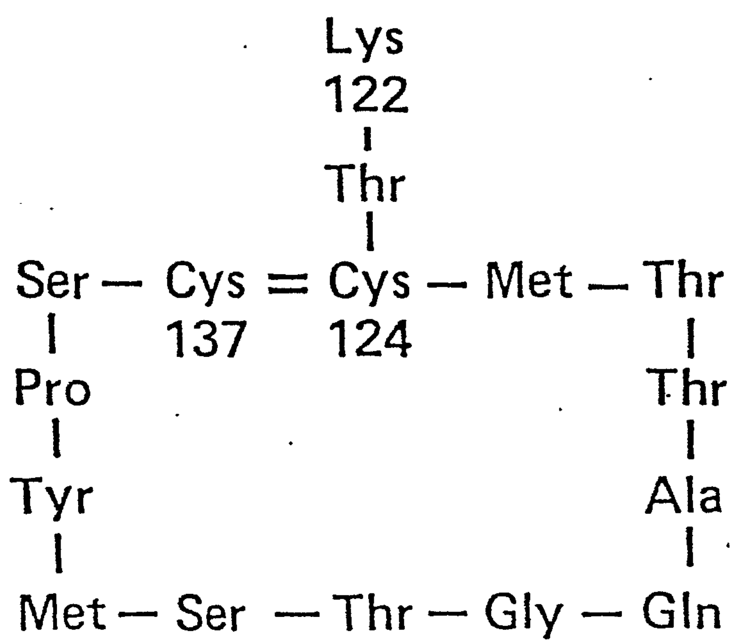

PEPTIDE 1 MW = 1708

FIGURE 3

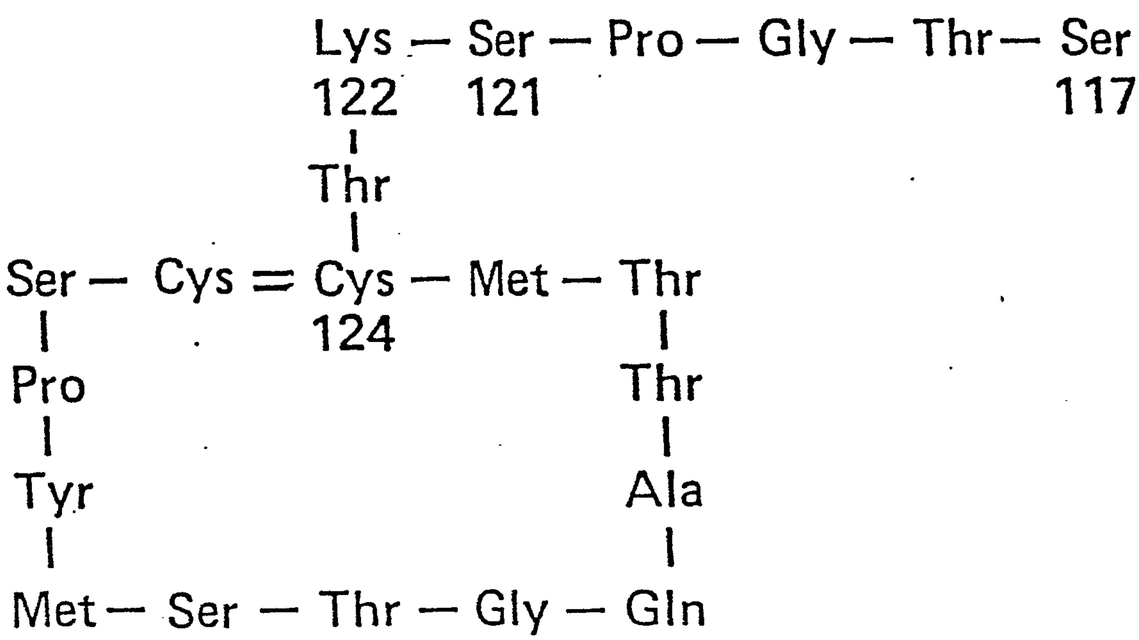

PEPTIDE 2 MW = 2137

FIGURE 4. Published Sequences of HBsAg P25

| | 116 | 117 | 118 | 119 | 120 | 121 | 122 | 123 | 124 | 125 | 126 | 127 | 128 | 129 | 130 | 131 | 132 | 133 | 134 | 135 |
|---|---|---|---|---|---|---|---|---|---|---|---|---|---|---|---|---|---|---|---|---|
| Reference 29 <u>ayw</u> | Thr | Ser | Thr | Gly | Pro | Cys | Arg | Thr | Cys | Met | Thr | Thr | Ala | Gln | Gly | Thr | Ser | Met | Tyr | Pro |
| Reference 30 assume <u>adw</u> | | | | | | | Lys | | | Thr | | Pro | | | | Asn | | | Phe | |
| Reference 31 <u>ady</u> | | | | | Ser | | | | | Thr | | Pro | | | | Ile | | | Thr | |
| Ref. 8b and 8c <u>adw</u> | | | | | | | | | | Thr | | Pro | | | | Asn | | | Phe | |
| <u>ayw</u> | | | | | | | | | | Thr | | Pro | | | | Thr | | | Tyr | |

| | 136 | 137 | 138 | 139 | 140 | 141 | 142 | 143 | 144 | 145 | 146 | 147 | 148 | 149 | 150 |
|---|---|---|---|---|---|---|---|---|---|---|---|---|---|---|---|
| Reference 29 <u>ayw</u> | Ser | Cys | Cys | Cys | Thr | Lys | Pro | Ser | Asp | Gly | Asn | Ser | Thr | Cys | Ile |
| Reference 30 assume <u>adw</u> | | | | | | | | Thr | | | | | | | |
| Reference 31 <u>ady</u> | | | | | | | | Ser | | | | | | | |
| Ref. 8b and 8c <u>adw</u> <u>ayw</u> | | | | | | | | | | | | | | | |

FIGURE 5

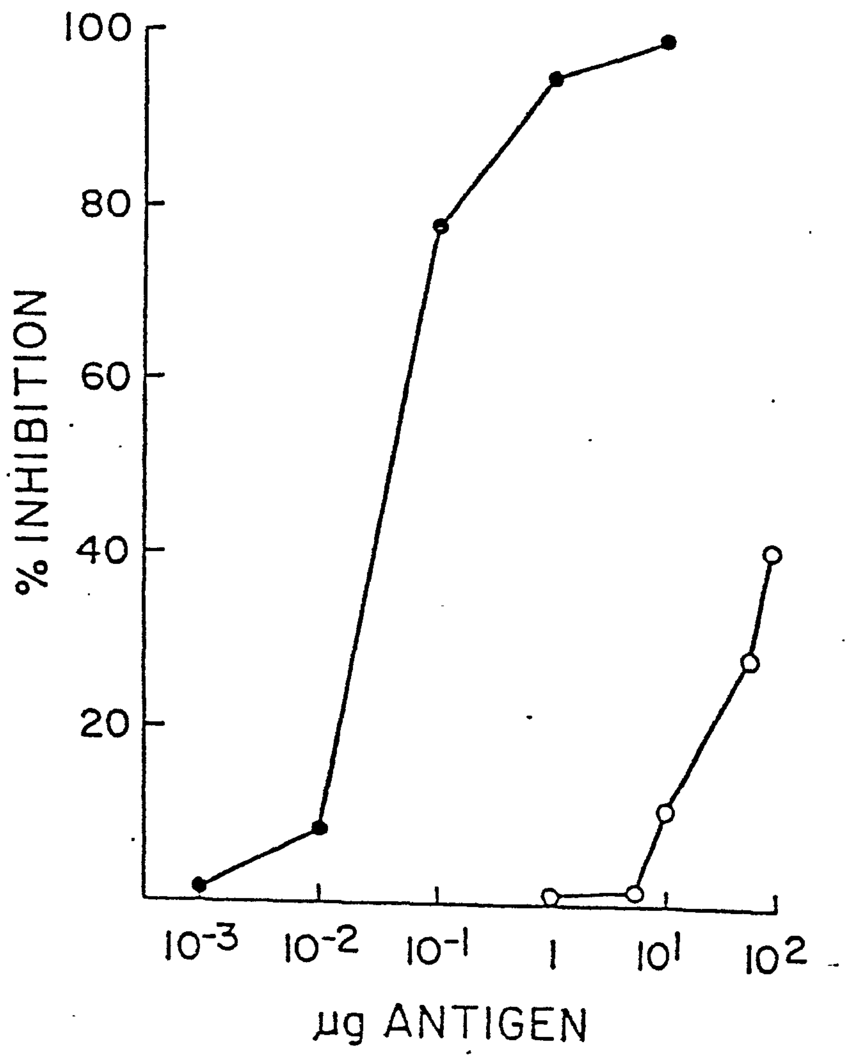


Competitive binding assay of an anti-a monoclonal antibody (A-12) with purified HBsAg (•) and with the cyclic peptide 1 (o). Micro wells coated with HBsAg/ade were incubated with a mixture of antibody and the respective antigen. The residual antibody activity bound to the HBsAg attached to the micro wells was detected by the addition of a $^{125}I$-labeled goat anti-mouse IgG reagent.

FIGURE 6

# IDIOTYPES

A. Reaction of idiotype with specific epitope (HBs peptide)

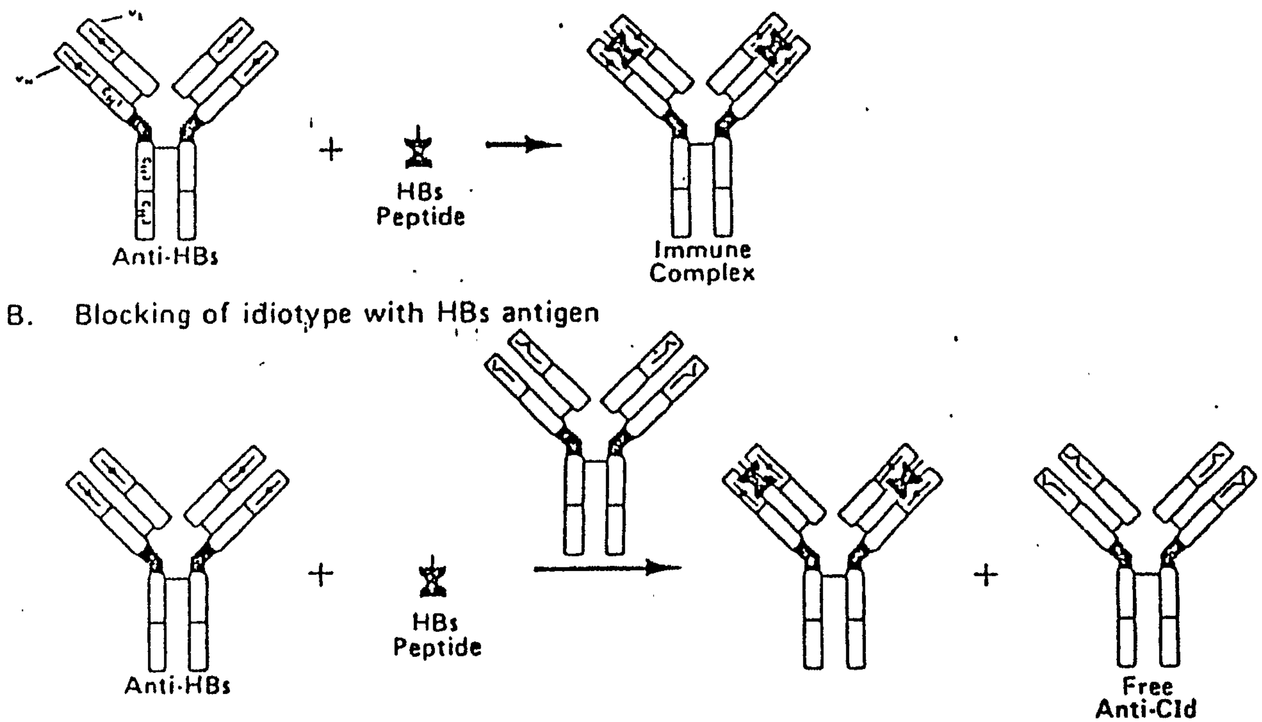


Illustration of the basic mechanism of the idiotype system. (A) The antigen-combining site of the antibody (—◆—) is defined as the idiotype that reacts with the specific HBs peptide ( ). (B) If the anti-HBs is preincubated with a specific antigen, a subsequent incubation with a rabbit anti-idiotypic antibody (√—) is blocked.

FIGURE 7

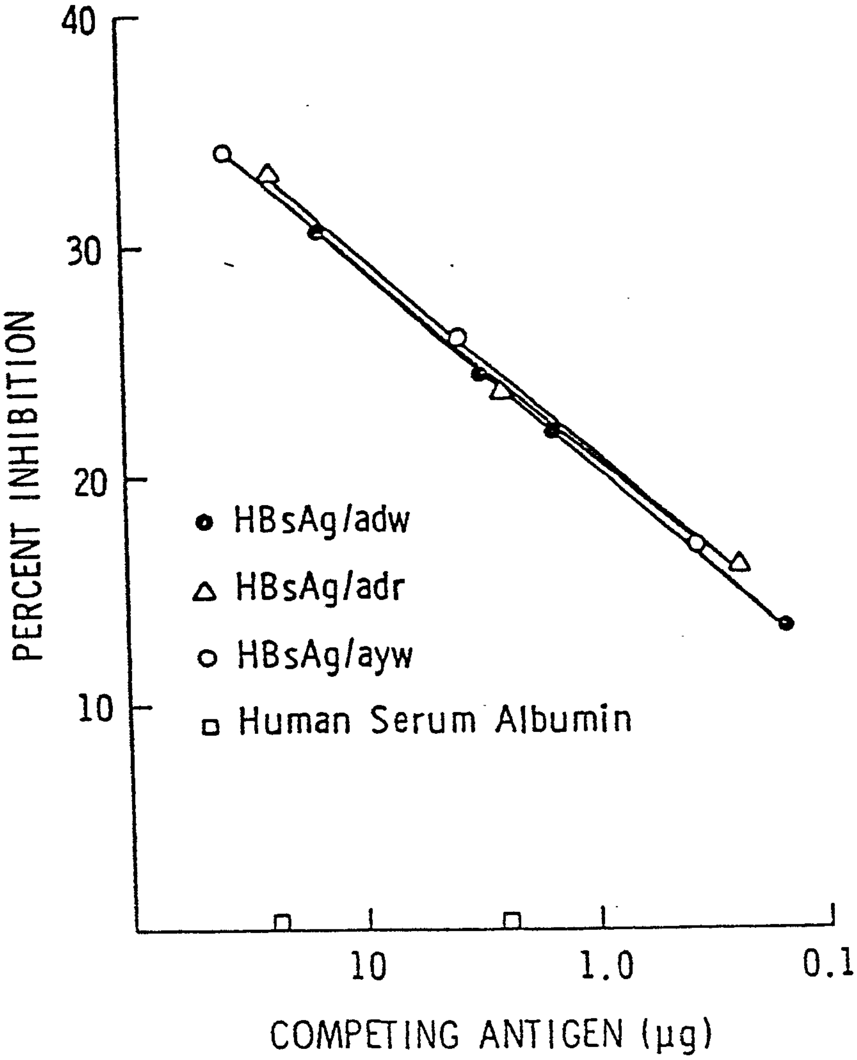
40
30
20
10
PERCENT INHIBITION
HBsAg/adw
HBsAg/adr
HBsAg/ayw
Human Serum Albumin
10
1.0
0.1
COMPETING ANTIGEN (μg)